# EUROPÄISCHE PATENTSCHRIFT

(11) **EP 4 179 977 B1**
(45) Veröffentlichungstag und Bekanntmachung des Hinweises auf die Patenterteilung: **03.09.2025**
(21) Anmeldenummer: 22207332.2
(22) Anmeldetag: 14.11.2022
(51) Int. Cl.: A61B 6/51, G03B 42/04

(54) **VORRICHTUNG ZUR HALTERUNG EINES SPEICHERMEDIUMS**
STORAGE MEDIUM HOLDING DEVICE
DISPOSITIF DE SUPPORT D'UN SUPPORT DE STOCKAGE

(30) Priorität: 15.11.2021 DE 102021129771
(43) Veröffentlichungstag der Anmeldung: 17.05.2023
(73) Patentinhaber: Dürr Dental SE, 74321 Bietigheim-Bissingen (DE)
(72) Erfinder: Kühfuß, Andreas, 71640 Ludwigsburg (DE); Mustajbegovic, Enes, 74321 Bietigheim-Bissingen (DE)
(74) Vertreter: Frenkel, Matthias Alexander

(56) Entgegenhaltungen:
- DE-T1- 19 581 556
- US-A1- 2004 170 253
- US-A1- 2005 053 199
- US-A1- 2017 202 526
- US-B1- 6 540 399

## Beschreibung

### Hintergrund der Erfindung

### 1. Gebiet der Erfindung

Die Erfindung betrifft eine Vorrichtung zur Halterung eines Speichermediums in der Mundhöhle eines Patienten für wenigstens eine Röntgenaufnahme, wobei die Vorrichtung einen Halterungsabschnitt und einen Aufbissabschnitt aufweist.

### 2. Beschreibung des Standes der Technik

Derartige Vorrichtungen sind Bestandteile von Haltesystemen für Speicherfolien. Ziel solcher Haltesysteme ist es, eine Speicherfolie im Mundraum des Patienten zu einer Röntgenvorrichtung auszurichten und zu halten.

Der übliche Aufbau eines solchen Haltesystems umfasst neben der Vorrichtung zur Halterung einer Speicherfolie mit Folienhalterungsabschnitt und Aufbissabschnitt zusätzlich eine an der Vorrichtung anbringbare Führungsstange, die auf einer der Haltevorrichtung abgewandten Seite zusätzlich einen Visierring aufweisen kann. Mittels der Führungsstange und gegebenenfalls des Visierrings kann die Ausrichtung der Röntgenvorrichtung sowie der Abstand eingestellt werden.

Für die Durchführung einer Röntgenaufnahme wird die Haltevorrichtung in den Mundraum des Patienten eingeführt. Dieser beißt zur Fixierung auf den Aufbissabschnitt der Haltevorrichtung, sodass das Haltesystem durch den, durch den Kiefer aufgebauten Druck beim Zusammenbeißen festgehalten und ausgerichtet bleibt.

Speichermedien wie beispielsweise Speicherfolien werden im Dentalbereich für unterschiedliche Aufnahmebereiche und -anforderungen wie beispielsweise Frontzahnaufnahmen, Bissflügelaufnahmen, etc. in unterschiedlichen Größen eingesetzt. Das eigentliche sogenannte intraorale Speichermedium befindet sich während der Röntgenaufnahme in einer Schutzhülle, die vor Lichteinfall oder/und möglicher Kontamination schützt.

Beispielsweise wird die Speicherfolie für das Auslesen der Bilddaten in einem Scanner von der Schutzhülle getrennt. Nach Abschluss der Bildinformationsauswertung wird die Speicherfolie gelöscht und anschließend wieder in eine neue hygienische Schutzhülle eingebracht; die bereits verwendete Hülle wird entsorgt. Durch das wiederholte Einbringen und Entnehmen der Speicherfolie in die/aus der Hülle bzw. Umhüllung muss ein gewisser Abstand zwischen Speicherfolie und Hülle bzw. zwischen Speichermedium im Allgemeinen und dessen Umhüllung vorhanden sein. Dies erschwert das exakte Positionieren der Speicherfolie mittels der bestehenden Haltesysteme.

Die WO 2018/022564 A1 beschreibt unter anderem einen Halter für eine Speicherfolie. Der Halter weist einen Aufbissblock sowie eine rechtwinklig dazu angeordnete Stützplatte auf. Die Stützplatte weist Federarme auf, die ein an einer Speicherfolienhülle angebrachtes Band straffen und so die Speicherfolie an die Stützplatte anziehen.

Nachteilig an diesem Haltesystem ist es, dass an der Schutzhülle der Speicherfolie ein zusätzliches Halteband benötigt wird. Dies bedeutet einen zusätzlichen Fertigungsschritt und erhöht somit die Herstellungskosten. Hinzu kommt, dass das Halteband gedehnt, bzw. überdehnt werden und reißen kann, so dass die Hülle gewechselt und die Speicherfolie ausgetauscht werden muss, was letztlich zu mehr Aufwand führt. Hiervon abgesehen zieht das Band bei Beanspruchung im Bereich der Naht an den die Hülle bildenden Folienseiten. Durch die Zugkraft des Bandes kann so die Naht verletzt werden, was zu einer unerwünschten Kontaminierung führen kann.

In der DE 8610962 U1 wird ein Haltesystem beschrieben, das eine Bissplatte sowie ein zu der Bissplatte rechtwinklig angeordnetes Rückschild aufweist. Das Rückschild ist an der Oberseite kreisbogenförmig umgebogen und übergreift einen daran eingesetzten Zahnfilm. Gegenüberliegend an der Unterseite ist eine federnd-elastische Klemmleiste ausgebildet, mittels der eine Klemmwirkung auf einen eingesetzten Zahnfilm ausgeübt wird. Beim Einsetzen eines Zahnfilms muss dieser vergleichsweise stark gebogen werden. Zudem ist die Klemmkraft der Klemmleiste festgelegt und kann nicht beispielsweise für das Einlegen oder Entnehmen des Zahnfilms aufgehoben werden.

Die US 7,033,075 B2 beschreibt ein Haltesystem, bei dem an einem Aufbissblock zwei Wangen angebracht sind, zwischen denen ein Sensor eingeklemmt werden kann. Die dem Bissblock abgewandte Wange kann mittels einer Schraube relativ zu der anderen Wange verstellt und so die Anpresskraft auf den Sensor hergestellt und eingestellt werden. Nachteilig an dieser Ausgestaltung ist die aufwändige Ausgestaltung der Mechanik und die damit auch einhergehende Schwierigkeit bei der Desinfektion. Hinzu kommt, dass diese Ausgestaltung ausschließlich für feste Sensoren im Unterschied zu Speicherfolien geeignet ist, da durch den manuell aufzubringenden Anpressdruck die Speicherfolie beschädigt werden könnte.

Ein anderes Haltesystem für Sensoren wird in der US 6 540 399 B1 gezeigt. Diese Vorrichtung umfasst einen Halteabschnitt und einen Aufbissabschnitt, ein erstes Teil mit einem ebenen Anlagebereich für den Sensor und ein zweites Teil mit einem Klemmbereich für den Sensor. Das erste Teil und das zweite Teil sind relativ zueinander zwischen einer Offenstellung und einer Klemmstellung bewegbar und in der Klemmstellung ist zwischen dem Anlagebereich und dem Klemmbereich eine Klemmkraft auf einen dazwischen befindlichen Sensor ausübbar, wobei das erste Teil und das zweite Teil in der Klemmstellung über ein Zusammenwirken des ersten und des zweiten Arretierbereichs zueinander arretierbar sind.

In der WO 2010/138684 A1 ist ebenfalls ein Haltesystem für Sensoren und nicht für Speicherfolien beschrieben, das zwei durch ein Gummiband verbundene Klemmbacken aufweist, zwischen denen ein Speichersensor angeordnet wird. Diese Vorrichtung ist ebenfalls nicht für Speicherfolien geeignet, da Speicherfolien nicht die für diese Art der Halterung erforderliche Steifigkeit aufweisen.

### ZUSAMMENFASSUNG DER ERFINDUNG

Es ist eine Aufgabe der Erfindung, eine Vorrichtung zur Haltung eines Speichermediums in der Mundhöhle eines Patienten anzugeben, die einfach zu desinfizieren ist und eine sichere und schonende Halterung des Speichermediums wie auch eine gewisse Bequemlichkeit für den Patienten ermöglicht. Insbesondere ist es wünschenswert, wenn auf das Speichermedium während des Röntgenaufnahmevorgangs eine definierte Klemmkraft aufgeprägt, die Klemmkraft aber für das Beladen der Halterung mit dem Speichermedium aufgehoben oder zumindest verringert werden kann. Das Entladen der Speicherfolie kann im geschlossenen Zustand durch Überwinden der an der Speicherfolie anliegenden Klemmkraft erfolgen.

Die Aufgabe wird durch eine Vorrichtung gemäß dem unabhängigen Anspruch gelöst. Weitere Ausgestaltungen der Erfindung sind in den abhängigen Ansprüchen angegeben.

Die erfindungsgemäße Vorrichtung zur Halterung eines Speichermediums in der Mundhöhle eines Patienten für wenigstens eine Röntgenaufnahme weist einen Halterungsabschnitt und einen Aufbissabschnitt, ein erstes Teil mit einem Anlagebereich mit einer eben ausgebildeten Anlagefläche für das Speichermedium, einem ersten Führungs- oder Gelenkbereich sowie einem ersten Arretierbereich und ein zweites Teil mit einem Klemmbereich für die Speicherfolie, wobei der Klemmbereich bezüglich der Speicherfolie eine konvex geformte Fläche aufweist, einen mit dem ersten Führungs- oder Gelenkbereich korrespondierenden zweiten Führungs- oder Gelenkbereich sowie einen zweiten Arretierbereich auf. Der Anlagebereich und der Klemmbereich wirken zusammen und bilden den Halterungsabschnitt. Der erste und der zweite Führungs- oder Gelenkbereich wirken so zusammen, dass das erste Teil und das zweite Teil relativ zueinander zwischen einer Offenstellung und einer Klemmstellung bewegbar sind. In der Klemmstellung ist auf ein zwischen dem Anlagebereich und dem Klemmbereich befindliches Speichermedium eine Klemmkraft ausübbar bzw. aufprägbar. Das erste Teil und das zweite Teil sind in der Klemmstellung über ein Zusammenwirken des ersten und des zweiten Arretierbereichs relativ zueinander arretierbar.

Die erfindungsgemäße Vorrichtung weist durch ihre Ausgestaltung mehrere Vorteile auf. Mittels der miteinander korrespondierenden und zusammenwirkenden Führungs- oder/und Gelenkbereiche kann eine Relativbewegung geführt stattfinden, so dass die auf ein Speichermedium ausgeübte Klemmkraft in der Klemmstellung anliegt und in der Offenstellung nicht oder nur mit geringer Klemmkraft anliegt. Dies ermöglicht ein gefahrloses und gegebenenfalls einhändiges Einbringen bzw. Entnehmen des Speichermediums in den Halterungsabschnitt der Vorrichtung. Durch die Arretiermöglichkeit, welche das erste Teil und des zweite Teil in der Klemmstellung relativ zueinander festlegt, kann eine vorher vorbestimmte Klemmkraft auf das Speichermedium ausgeübt werden. Dies minimiert Fehlereinflüsse bei der Erstellung einer Röntgenaufnahme, die durch Fehlpositionierungen oder durch lokale Beschädigungen des Speichermediums im Klemmbereich auftreten könnten. Durch den zweiteiligen Aufbau kann vorgesehen sein, dass die Teile voneinander separierbar sind und dadurch einfach für eine Sterilisation zugänglich sein können.

Bei einer Ausführungsform der Erfindung kann der Halterungsabschnitt ergonomische Bereiche aufweisen, die ein sicheres Halten des ersten Teils zwischen Zeige- und Mittelfinger eines Anwenders ermöglicht, wobei mittels wiederum ergonomischem Anliegen des Daumens eines Anwenders die Vorrichtung in die Klemmstellung gebracht werden kann.

Bei einer weiteren Ausführungsform der Erfindung ist vorgesehen, dass der Anlagebereich eine im Wesentlichen flächig ausgebildete Anlagefläche zur flächigen Abstützung eines Speichermediums aufweist. Die Anlagefläche unterstützt somit eine knick- und krümmungsfreie Positionierung des Speichermediums während der Röntgenaufnahme.

Die auf das Speichermedium einwirkende Klemmkraft wird durch die Formgebung des Klemmbereichs bevorzugt linienförmig aufgebracht, was in vorteilhafter Weise ein einfaches Herauslösen des Speichermediums aus der Haltevorrichtung ermöglicht, ohne diese zu diesem Zeitpunkt vollständig zu demontieren. Die Haltevorrichtung kann nach dem Durchführen der Röntgenaufnahme und der Entnahme des Speichermediums in einem Behälter zwischengelagert und zu einem späteren Zeitpunkt für eine Desinfektion zerlegt werden.

Vorteilhaft kann vorgesehen sein, dass die Anlagefläche für das Speichermedium in einem Winkel zwischen 75° und 95°, bevorzugt zwischen 80° und 90° zu dem Aufbissabschnitt angeordnet ist. Eine Vorneigung zwischen 80° und 90° unterstützt eine Positionierung des Speichermediums möglichst nah an dem zu röntgenden Bereich. Dabei kann es sich beispielsweise in nicht abschließender Aufzählung um einen Zahnbereich, einen Kieferbereich oder einen Implantatbereich handeln. In einer bevorzugten Ausführungsform kann eine Verrastung, beispielsweise ein in ein Zahnrad eingreifender Rastzapfen, vorgesehen sein, die eine individuelle Einstellbarkeit der Neigung der Anlagefläche für die gewünschte Aufnahme zulässt.

Bei einer weiteren Ausführungsform der Erfindung kann vorgesehen sein, dass der Halteabschnitt in Bezug auf den Aufbissabschnitt in seiner Neigung verstell- und arretierbar ist. Somit kann eine individuelle Einstellung des Neigungswinkels zwischen dem Halteabschnitt und dem Aufbissabschnitt vorgenommen und die Haltevorrichtung besser an die Gegebenheiten in der Mundhöhle angepasst werden.

Bevorzugt ist die Neigung in einem Winkel zwischen 75° und 95°, bevorzugt zwischen 80° und 90°, zu dem Aufbissabschnitt anordenbar oder/und einstellbar.

Bei einer weiteren bevorzugten Ausführungsform sind an dem ersten Teil und dem zweiten Teil in dem Arretierbereich miteinander korrespondierende Ausnehmungen so angeordnet, dass durch die Ausnehmungen eine Arretiervorrichtung steckbar und so das erste Teil und das zweite Teil miteinander arretierbar sind. Bevorzugt sind dazu die Ausnehmungen gleich oder sehr ähnlich dimensioniert und fluchten miteinander für den Arretiervorgang. Bei der Arretiervorrichtung kann es sich beispielsweise um Abschnitte einer Zielstange handeln. Auf diese Weise kann die Haltevorrichtung in der Klemmstellung verriegelt bzw. arretiert werden.

Es kann mittels der Haltevorrichtung eine definierte Kraft von dem Klemmbereich auf den Anlagebereich ausgeübt und so eine vorbestimmte Klemmkraft erzeugt werden. Die so erzeugte Klemmkraft ist hierbei diejenige Kraft, die aufgewendet werden muss, um ein von der Haltevorrichtung gehaltenes Speichermedium ohne ein Lösen der Klemmstellung herauszuziehen. Diese Klemmkraft kann bei einer Ausführungsform beispielsweise im Bereich von 1,8 N bis 4,2 N, vorzugsweise in einem Bereich zwischen 2,2 N und 3,4 N und besonders bevorzugt in einem Bereich zwischen 2,5 N und 3,0 N liegen. Eine Klemmkraft in dieser Größenordnung verhindert sicher ein Verschieben oder ein Herausfallen des Speichermediums. Wie vorangehend beschrieben kann das Speichermedium (mit einer Umhüllung wie beispielsweise eine umhüllende Schutzfolie oder ein Schutzschlauch) nach dem Durchführen einer Röntgenaufnahme durch Überwinden der Klemmkraft aus der Haltevorrichtung beispielweise zum Auslesen der gespeicherten (Bild-)Daten herausgezogen, ohne die Haltevorrichtung zu demontieren oder die Klemmstellung zu lösen.

Auf die tatsächliche Höhe der Klemmkraft haben eine Reihe von Faktoren Einfluss: Beispielsweise kann durch Feuchtigkeit im Mundraum auf die Oberflächen des Speichermediums bzw. der Umhüllung, des Anlagebereichs oder/und des Klammbereich während des Einbringens oder/und während der Röntgenaufnahme aufgebrachte Flüssigkeit die Oberflächenreibung verändert werden; die durch die Haltevorrichtung auf das Speichermedium in der Klemmstellung aufgeprägten Kraft kann durch Fertigungstoleranzen unterschiedlich oder durch Materialbruch oder/und Materialdehnung im Betrieb verändert werden.

Auch ist die Höhe der Klemmkraft bei einem Messvorgang durch die Geschwindigkeit des Herausziehens und dabei stattfindendem Schlupf oder/und Formänderungsschlupf sowie durch ein teilweises Gleiten beinflussbar.

Bei einer Ausgestaltung weist die Arretiervorrichtung eine Zielvorrichtung für das Ausrichten einer Röntgenvorrichtung auf.

Bei einer weiteren Ausführungsform weist der Führungsbereich eine Leistenführung auf, die so ausgestaltet ist, dass der Anlagebereich und der Klemmbereich relativ zueinander verschiebbar und so die Klemmkraft von dem Klemmbereich auf den Anlagebereich, insbesondere bei einem dazwischen befindlichen Speichermedium, ausübbar ist. Die Leisten-führung kann beispielsweise als Nut-Leisten-Führung ausgebildet sein. Eine solche Leistenführung ist technisch einfach zu realisieren, erlaubt eine einfache Trennung von erstem Teil und zweitem Teil zu Sterilisationszwecken und ermöglicht zudem eine einfache Einhandbedienung der Vorrichtung.

Alternativ zu der relativen Verschiebbarkeit von Anlagebereich und Klemmbereich kann der Gelenkbereich an dem ersten oder dem zweiten Teil eine Drehachse und eine dazu korrespondierende Lagerung an dem anderen Teil aufweisen.

Bevorzugt ist die Drehachse so angeordnet, dass der Anlagebereich und der Klemmbereich eine Schwenkbewegung relativ zueinander ausführen. Dies ermöglicht ebenfalls die Realisierung einer Klemm- und Offenstellung. In Kombination mit einer Festlegung der beiden Teile zueinander im Arretierbereich kann so ebenfalls eine definierte Anlagekraft von 1,8 N bis 4,2 N, vorzugsweise in einem Bereich zwischen 2,2 N und 3,4 N und besonders bevorzugt in einem Bereich zwischen 2,5 N und 3,0 N auf das Speichermedium sowie eine Entnahme des Speichermediums durch Überwinden der Klemmkraft, wie vorangehend bereits beschrieben, realisiert werden.

Insgesamt ist es vorteilhaft, wenn zwischen dem Anlagebereich und dem Klemmbereich ein Abstand vorhanden ist, der so dimensioniert ist, dass in der Offenstellung ein Speichermedium zwischen dem Anlagebereich und dem Klemmbereich eingebracht werden kann und der Klemmstellung eine vorbestimmte Klemmkraft auf das Speichermedium ausgeübt wird.

Bei einer Ausführungsform können je nach einzubringendem Speichermedium der Abstand zwischen Anlagebereich und Klemmbereich variierbar sein.

Gleichzeit oder alternativ kann die angelegte Klemmkraft, beispielsweise in den zuvor genannten Bereichen, variierbar sein. Die Realisierung der notwendigen Klemmkraft wie auch die Fixierung der Vorrichtung wird durch die erfindungsgemäß angeordneten Arretierbereiche, in welche die Arretiervorrichtung in abhängig von der Größe des Speichermediums eingesetzt werden kann, sichergestellt.

Der Begriff Speichermedium soll im Rahmen der Anmeldung vorrangig eine Speicherfolie und die Schutzhülle, in der sich die Speicherfolie für wenigstens eine Röntgenaufnahme befindet, mit umfassen. Je nach vorliegender Ausführungsform soll jedoch auch eine Anwendung der Erfindung auf einen Speicherchip, einen Speichersensor und einen Speicherfilm sowie gegebenenfalls zusätzlich die jeweilige Schutzumhüllung vom Schutzumfang mit umfasst sein.

### KURZE BESCHREIBUNG DER ZEICHNUNGEN

Nachfolgend werden Ausführungsbeispiele der Erfindung anhand der Zeichnungen näher erläutert. In diesen zeigen:
- Figur 1: eine perspektivische Ansicht einer ersten Ausführungsform eines Speichermediumhalters mit Basisteil und Schiebeteil in einer Offenstellung;
- Figur 2: eine Seitenansicht der Ausführungsform der Figur 1;
- Figur 3: die Seitenansicht der Figur 2, bei der sich Basisteil und Schiebeteil in einer Klemmstellung befinden;
- Figuren 4, 5: eine perspektivische und eine Seitenansicht des Basisteils des Speichermediumhalters der Figuren 1-3;
- Figuren 6, 7: eine perspektivische und eine Seitenansicht des Schiebeteils des Speichermediumhalters der Figuren 1-3;
- Figur 8: eine perspektivische Ansicht der ersten Ausführungsform in einer Klemmstellung mit einer angesetzten Arretiervorrichtung;
- Figur 9, 10: Seitenansichten einer zweiten Ausführungsform in Offenstellung und Klemmstellung; und
- Figuren 10, 11: eine Seitenansicht sowie eine Draufsicht einer Ausführungsform eines Speichermediumhalters in einer Offenstellung.

### BESCHREIBUNG BEVORZUGTER AUSFÜHRUNGSBEISPIELE

### Erstes Ausführungsbeispiel

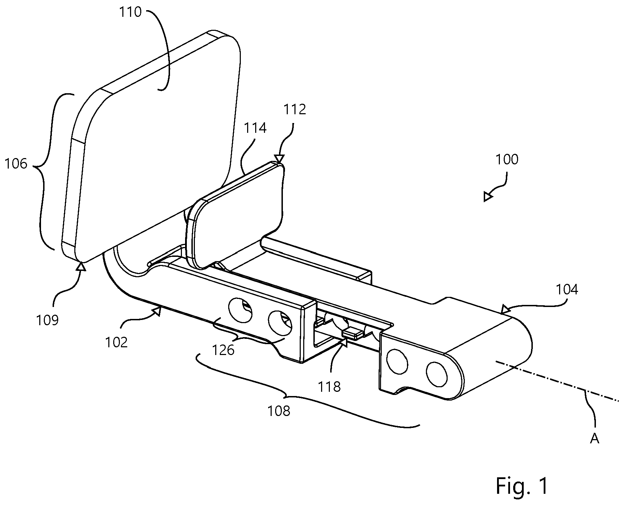

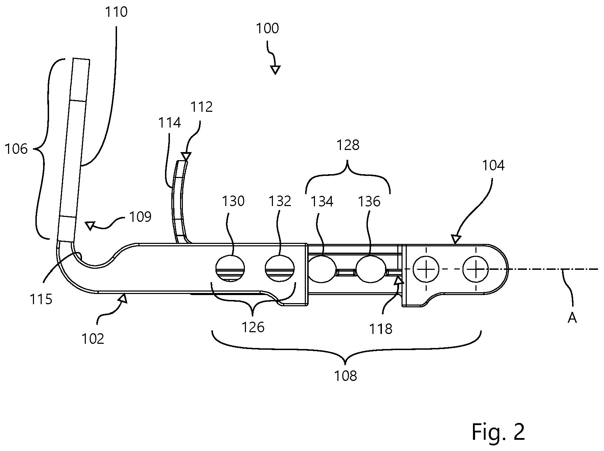

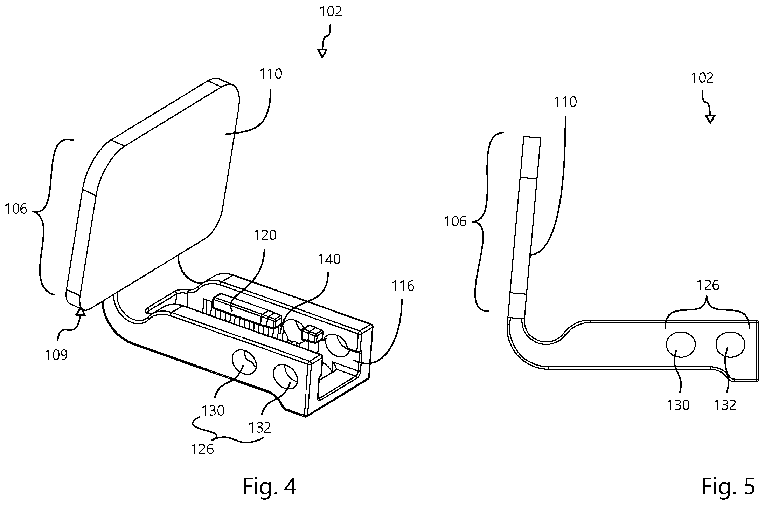

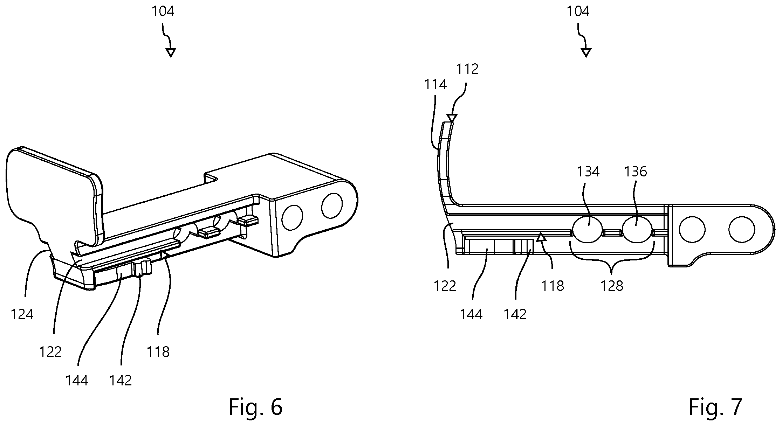

Die Figuren 1 und 2 veranschaulichen eine erste erfindungsgemäße Ausführungsform eines Speichermediumhalters 100. Der Speichermediumhalter 100 umfasst ein Basisteil 102 sowie ein Schiebeteil 104. Das Basisteil 102 ist separat in einer perspektivischen und einer Seitenansicht in den Figuren 4, 5, dargestellt, das Schiebeteil 104 in den Figuren 6, 7.

Der Speichermedienhalter 100 weist einen Halterungsabschnitt 106 sowie einen Aufbissabschnitt 108 auf. Der Halterungsabschnitt 106 wird seitens des Basisteils 102 durch einen Anlagebereich 109 mit einer flächig ausgebildeten Anlagefläche 110, seitens des Schiebeteils 104 durch einen Klemmbereich 112 gebildet. Die eben ausgebildete Anlagefläche 110 ist so ausgestaltet, dass sie ein Speichermedium - in der Regel in einer Schutzhülle befindlich - abstützen kann, so dass diese die vorgesehene Form - in der Regel eben - einnehmen kann.

Der Klemmbereich 112 weist, wie gut in Figur 2 zu erkennen ist, eine, bezüglich des Anlagebereichs 109 konvex geformte Klemmfläche 114 auf. In der gezeigten Ausführungsform ist die Klemmfläche 114 generell zylinderförmig ausgestaltet, wobei die Längsachse des Krümmungszylinders im Wesentlichen parallel zu der Anlagefläche 110 verläuft. Auf diese Weise kann entlang der Zylinderachse von der Klemmfläche 114 auf die Anlagefläche 110 bzw. auf ein dazwischen befindliches Speichermedium (nicht gezeigt) eine Anpresskraft gleichförmig aufgeprägt werden.

Der Aufbissabschnitt 108 erstreckt sich im Wesentlichen senkrecht zu der Anlagefläche 110 entlang einer Achse A. Der Winkel, den die Anlagefläche 110 und die Achse A einnehmen, kann beispielsweise zwischen 75° und 95°, bevorzugt zwischen 80° und 90°, liegen.

An der Stelle, an der die Anlagefläche 110 des Halterungsabschnitts 106 in den Aufbissabschnitt 108 übergeht, ist ein konkav geformter Übergangsabschnitt 115 vorgesehen bzw. an- oder ausgeformt. Dieser ist so gestaltet, dass ein in den Bereich zwischen dem Anlagebereich 109 und dem Klemmbereich 112 eingebrachtes Speichermedium, insbesondere eine Speicherfolie, nicht mit der beim Einführvorgang vorne befindlichen Kante hart gegen eine Oberfläche stößt, sondern sanft abgleiten kann und so beispielsweise eine Beschädigung der Kante des Speichermediums - wie es beispielsweise bei einer vergleichsweise empfindliche Speicherfolie oder der sie umhüllenden Schutzhülle der Fall sein könnte - vermieden werden kann.

Das Basisteil 102 weist im Bereich des Aufbissabschnitt 108 einen ersten Führungsbereich 116, das Schiebeteil einen korrespondierenden zweiten Führungsbereich 118 auf - siehe hierzu die Figuren 4 und 6. Die Führungsbereiche 116, 118 wirken in der gezeigten Ausführungsform ähnlich einer Schubladenführung zusammen: An dem Basisteil 102 sind im ersten Führungsbereich 116 zwei Leisten 120 (in den Figuren ist nur eine Leiste 120 erkennbar, siehe z.B. Fig. 4) angebracht, die einander gegenüberliegen und in Nuten 122, 124 des zweiten Führungsbereichs 118 des Schiebeteils 104 eingreifen. Die Leisten 120 und die zugehörigen Nuten 122, 124 sind in der gezeigten Ausführungsform teilweise unterbrochen ausgeführt, um eine einerseits Raum für eine nachstehend beschriebene Arretiervorrichtung zu schaffen und um gleichzeitig möglichst über die gesamte Länge des Bewegungsumfangs des Basisteils 102 und des Schiebeteils 104 eine Führung durch die Nut-Leisten-Führung zu schaffen.

Die Nut-Leistenführung verläuft in der gezeigten Ausführungsform parallel zu der Längsachse A des Aufbissabschnitts 108. Dies ist aber nicht notwendig, es könnten auch ein Winkelversatz oder/und eine oder mehrere Unterbrechungen zwischen der Längsachse A und der Nut-Leistenführung bestehen (in den Figuren nicht gezeigt).

Mittels der beschriebenen Nut-Leistenführung wird eine Verschiebbarkeit zwischen dem Basisteil 102 und dem Schiebeteil 104 realisiert. Das Schiebeteil 104 kann so entlang einer Schubachse - hier: parallel zu der Längsachse A - relativ zu dem Basisteil 102 verschoben werden. Damit kann die Speichermediumhalterung 100 von einer Offenstellung in eine Klemmstellung und umgekehrt gebracht werden.

Die Offenstellung ist in den Figuren 1 und 2 dargestellt. Dort befindet sich zwischen der Anlagefläche 110 und der Klemmfläche 114 ein Abstand, der ein einfaches Einlegen des Speichermediums ermöglicht. Je nach Art des Speichermediums - Speicherfolie, Speichersensor, Speicherchip, Speicherfilm etc. - kann der Abstand für das Einlegen geeignet dimensioniert sein.

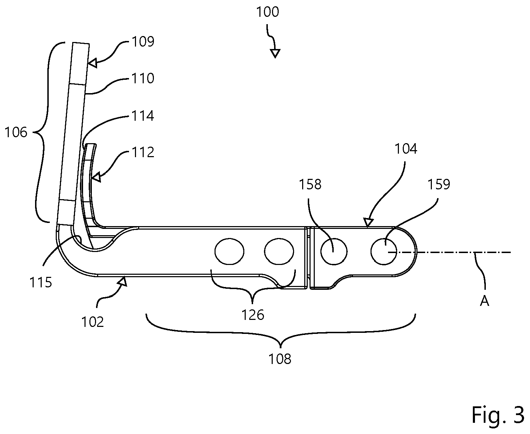

In der Klemmstellung hingegen, die in der Figur 3 veranschaulicht ist, ist das Schiebeteil 104 maximal in das Basisteil 102 eingeschoben. Entsprechend ist der Abstand zwischen der Anlagefläche 110 und der Klemmfläche 114 derart, dass das eingelegte Speichermedium mit einer Anlagekraft oder Anpresskraft beaufschlagt wird. Auf ein zwischen den beiden Flächen befindliches Speichermedium wird eine vorbestimmte Anlagekraft derart ausgeübt, dass eine Klemmkraft, insbesondere in einem Bereich von 1,8 N bis 4,2 N, vorzugsweise in einem Bereich zwischen 2,2 N und 3,4 N und besonders bevorzugt in einem Bereich zwischen 2,5 N und 3,0 N entsteht. Diese Anlagekraft bewirkt einerseits, dass das Speichermedium sich an die Anlagefläche 110 anlegt und andererseits aufgrund der entstehenden Klemmkraft gegen ein Verrutschen oder Herausfallen gesichert ist.

Um die gewünschte Anlagekraft und die daraus resultierende Klemmkraft zu erzielen, ist der Haltevorrichtung 100 auf eine bestimmte Dicke des Speichermediums und auf die darauf maximal applizierbare Anlagekraft abgestimmt bzw. abstimmbar. Beispielhafte Dicken der Speichermedien liegen zwischen 200 µm und 1000 µm, insbesondere zwischen 300 µm und 900 µm und beispielsweise bei 340 ± 40 µm oder 760 ± 80 µm für Speicherfolien sowie zwischen 5 mm und 8 mm, insbesondere zwischen 5,5 mm und 7,5 mm und beispielsweise bei 6,4 mm für Speichersensoren.

Um das Basisteil 102 und das Schiebeteil 104 gegeneinander gegen ein Verrutschen in der Klemmstellung zu sichern bzw. zu arretieren, sind jeweils an dem Basisteil 102 und an dem Schiebeteil 104 wenigstens ein Arretierbereich 126, 128 vorgesehen. Die Arretierbereiche 126, 128 sind jeweils durch ein Paar Öffnungen 130, 132 / 134, 136, hier beispielhaft als Bohrungen ausgeführt, realisiert, die in der vorgesehenen Klemmstellung miteinander fluchten. Die Öffnungen 130-136 sind in der gezeigten Ausführungsform zylindrisch ausgeführt. Es sind aber auch anderen Formgebungen wie beispielsweise, in nicht abschließender Aufzählung, mit einem quadratischen, dreieckigen, trapezförmigen oder elliptischen Querschnitt möglich.

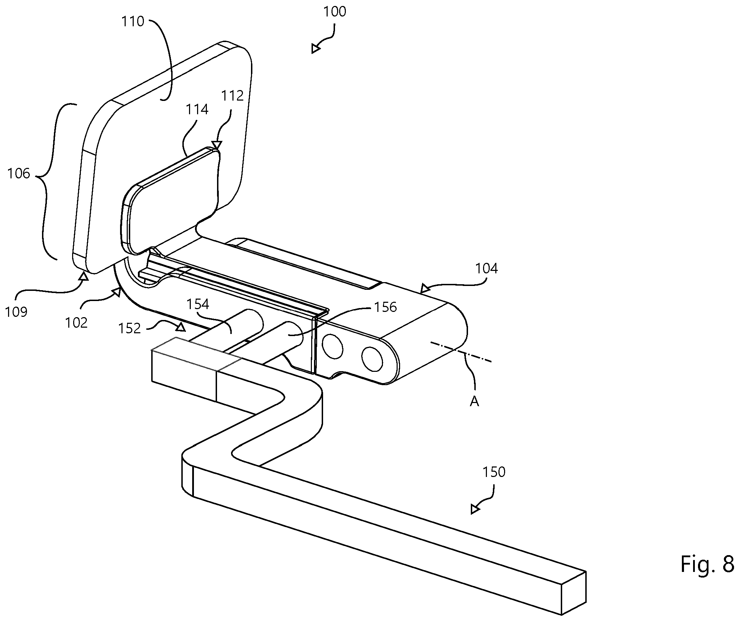

Wenn die Öffnungen 130, 132 des Arretierbereichs 126 des Basisteils 102 mit den Bohrungen 134, 136 des Arretierbereichs 128 des Schiebeteils 104 fluchten, kann in die so entstandenen fluchtenden Ausnehmungen eine Arretiervorrichtung 152 (siehe Figur 8) eingebracht, beispielsweise eingesteckt, werden. Bei der Arretiervorrichtung kann es sich beispielsweise um eine Ziel- oder Führungsstange 150 handeln, an welche eine Arretiervorrichtung 152 angeformt sein kann. Da die Arretierbereiche 126, 128 paarig angelegt sind, kann so eine räumliche Fixierung der Zielstange 150 mit einfachen Mitteln erreicht werden. Dies wäre allerdings auch alleine durch eine geeignete Formgebung der Öffnungen wie bereits erwähnt (quadratisch, dreieckig, trapezförmig, elliptisch, o. ä.) mit nur einer fluchtenden Ausnehmung möglich.

In einer alternativen Ausführungsform kann neben der eben beschriebenen Arretiermöglichkeit über die Arretierbereiche 126, 128 eine Verriegelung des Basisteils 102 mit dem Schiebeteil 104 über Rastnasen oder ähnliches erreicht werden. Eine solche Arretiervorrichtung ist in den Figuren 4 und 5 zu erkennen. In einem seitlichen Bereich - hier im Ausführungsbeispiel unterhalb der Leisten 120 - ist an dem Basisteil 102 eine Rastfläche 140 angebracht. Diese kann beispielsweise einzelne hervorstehende Rippen, Vertiefungen oder beispielsweise eine wellig geformte Oberfläche aufweisen. Die Rastfläche 140 korrespondiert mit einer oder mehreren Rastnocken 142 an dem Schiebeteil 104. Die Formgebung der Rastnocke 142 wirkt mit der Oberflächen-Formgebung der Rastfläche 140 zusammen, um die gewünschte Rastwirkung zu erreichen. Zusätzlich kann die Rastnocke 142 federnd ausgeführt sein. Dies ist in Figur 6 zu erkennen. Die Rastnocke 142 ist am Ende eines Federarms 144 angebracht und kann sich so bei einem Gleiten über die Oberfläche der Rastfläche 140 bei einem Einrasten in eine Vertiefung in Richtung der Oberfläche der Rastfläche 140 bewegen und bei einem Überwinden einer Erhöhung von dieser wegschwingen unter Überwindung der Federkraft des Federarms 144.

Figur 8 veranschaulicht die oben beschriebene Ausführungsform eines Speichermediumhalters 100 in einer perspektivischen Ansicht in der Klemmstellung, d.h. das Schiebeteil 104 ist vollständig in das Basisteil 102 entlang der Schiebeachse A eingeschoben. In dieser Stellung ist mittels der Arretiervorrichtung Rastnasen 142 / Rastfläche 140 bereits eine gewisse relative Arretierung des Basisteils 102 mit dem Schiebeteil 104 realisiert.

Um jedoch für die Handhabung in der Mundhöhle des Patienten eine absolut sichere Verriegelung der beiden prinzipiell zueinander verschieblichen Teile 102, 104 zu erreichen, kann eine wie bereits weiter oben beschriebene Arretierung mittels einer an einer Zielstange 150 angebrachten Arretiervorrichtung 152 erfolgen. Die Anordnung der Arretiervorrichtung 152 an der Zielstange 150 bzw. die Kombination von Arretiervorrichtung 152 und Zielstange 150 ist im Hinblick auf die gesamte Vorrichtung zwar vorteilhaft, es kann jedoch prinzipiell eine Arretiervorrichtung 152 auch ohne Zielstange 150 eingesetzt werden.

Die Arretiervorrichtung umfasst wie bereits oben angedeutet wenigstens einen Arretierstift, der geometrisch so geformt ist, dass er in Eingriff mit wenigstens eine der Öffnungen 130, 132 / 134, 136 gebracht werden kann. In dem in Figur 8 gezeigten Ausführungsbeispiel sind zwei zylindrische Arretierstifte 154, 156 vorgesehen, welche in die Öffnungen 130, 132 des Arretierbereichs 126 des Basisteils 102 und nachfolgend auch in die Bohrungen 134, 136 des Arretierbereichs 128 des Schiebeteils 104 eingeschoben werden können. Solange diese Arretierstifte 154, 156 in den Bohrungen der Arretierbereiche 126, 128 verbleiben, ist eine Relativbewegung der Teile 102, 104 zueinander nicht möglich, so dass auch ein zwischen der Anlagefläche 110 und der Klemmfläche 114 befindliches Speichermedium wie beispielsweise eine Speicherfolie (in der Regel mit Schutzhülle) sicher gehalten bzw. geklemmt werden kann.

Zusätzlich sind an dem Schiebeteil 104 weitere Öffnungen 158, 159 vorgesehen (siehe Figur 3). Beispielsweise kann die dem Basisteil 102 näher liegende Öffnung 158 ebenfalls zur Aufnahme eines Arretierstifts 154 vorgesehen sein, um eine Arretierung von Basisteil 102 und Schiebeteil 104 zu erreichen. Die weitere Öffnung 159 kann zusammen mit der Öffnung 158 eine Aufnahme der Arretierstifte 154, 156 der Zielstange 150 ermöglichen, ohne zu einer Arretierung von Basisteil 102 und Schiebeteil 104 zu führen.

### Zweites Ausführungsbeispiel

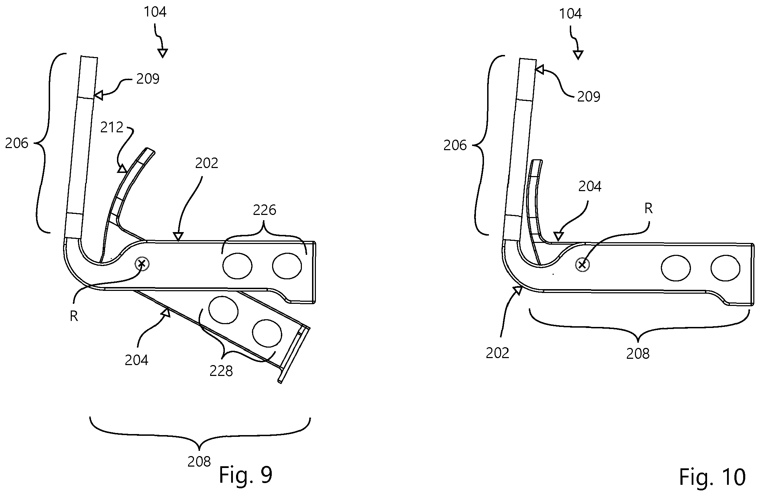

Die Figuren 9 und 10 veranschaulichen in Seitenansichten eine zweite Ausführungsform eines Speichermediumhalters 200. Der Speichermediumhalter 200 weist ein Basisteil 202 und ein Schwenkteil 204 auf. Die Figur 9 zeigt den Speichermediumhalter 200 in einer Offenstellung und die Figur 10 in einer Klemmstellung.

In Analogie zu dem Speichermediumhalter 100, wie er in den Figuren 1-8 veranschaulicht ist, weist der Speichermediumhalter 200 der Figuren 9 und 10 einen Halterungsabschnitt 206 sowie einen Aufbissabschnitt 208 auf. Diesbezüglich ist der Speichermediumhalter 200 weitgehend analog zu dem Speichermediumhalter 100 der Figuren 1-8 aufgebaut, so dass auf eine erneute Beschreibung gleicher oder sehr ähnlicher Merkmale verzichtet wird. Stattdessen werden in den Figuren und in der nachstehenden Beschreibung für gleiche oder sehr ähnlich Merkmale Bezugszeichen verwendet, zu denen 100 addiert wurde.

Im Unterschied zu dem Speichermediumhalter 100 der Figuren 1-8 erfolgt bei dem Speichermedienhalter 200 der Figuren 9 und 10 die Relativbewegung für einen Wechsel zwischen der Offenstellung der Figur 9 und der Klemmstellung der Figur 10 über eine Schwenk- bzw. Drehbewegung, bei der das Schwenkteil 204 um eine Achse R rotiert. Die Achse kann konstruktiv als umlaufende oder feststehende Achse, als separates (drittes Bauteil) oder als angeformter Achsabschnitt an dem Basisteil 202 oder dem Schwenkteil 204 ausgeführt sein. Auch können Basisteil 202 und Schwenkteil 204 fest über die Drehachse miteinander verbunden sein. Alternativ können Basisteil 202 und Schwenkteil 204 in einer bestimmten Drehstellung zueinander - beispielsweise in der Offenstellung - ineinander steckbar und nach einem bestimmten Drehwinkel miteinander verbunden sein.

### Drittes Ausführungsbeispiel

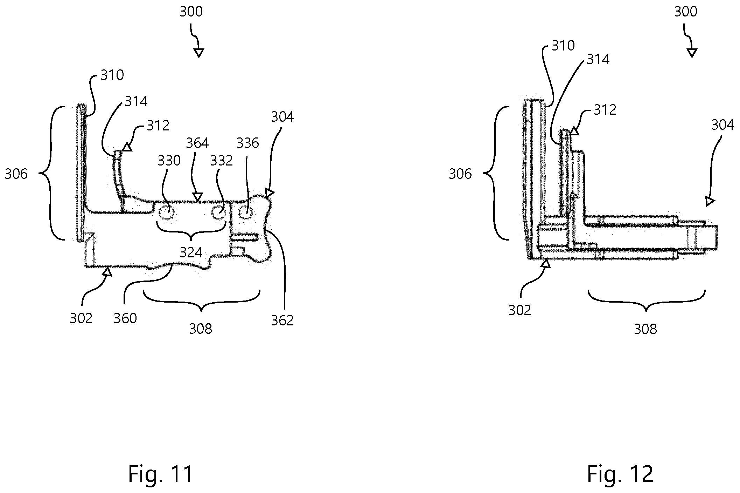

Die Figuren 11 und 12 veranschaulichen in einer Seitenansicht und in einer Draufsicht eine dritte Ausführungsform eines Speichermediumhalters 300. Der Speichermediumhalter 300 weist ebenfalls wie das erste Ausführungsbeispiel ein Basisteil 302 und ein Schiebeteil 304 auf. Aufgrund der Vielzahl gleicher oder vergleichbarer Merkmale wird auf eine erneute Beschreibung derselben verzichtet, um Wiederholungen zu vermeiden. Stattdessen werden gleiche oder vergleichbare Merkmale mit Bezugszeichen versehen, zu denen - verglichen mit dem ersten Ausführungsbeispiel - 200 addiert wurde.

Der Speichermediumhalter 300 weist - kurz zusammengefasst - ebenfalls einen Halterungsabschnitt 306 auf, der eine flächige Anlagefläche 310 am Basisteil 302 sowie einen Klemmbereich 312 mit einer konvex geformten Klemmfläche 314 am Schiebeteil 304 aufweist. Ebenso ist an dem Speichermediumhalter 300 ein Aufbissabschnitt 308 vorgesehen. An dem Aufbissabschnitt 308 ist am Basisteil 302 ein ergonomisch geformter erster Bereich 360 vorgesehen. Dieser korrespondiert mit einem zweiten ergonomischen Bereich 362, der stirnseitig an dem Schiebeteil 304 angeformt ist.

Für die Aufnahme bzw. Halterung eines Speichermediums in dem Halterungsabschnitt 306 kann eine Bedienperson den Speichermediumhalter 300 beispielsweise mit ihrer linken Hand so greifen, dass ihr Mittelfinger in dem ersten ergonomischen Bereich 360 zu liegen kommt, der Zeigefinger diesem gegenüberliegend auf der Oberseite 364 des Speichermediumhalters 300 und der Daumen an dem zweiten ergonomischen Bereich 362 anliegt. Die Bedienperson kann dann das Speichermedium mit der rechten Hand in den Halterungsabschnitt 306 einbringen und durch Drücken des Daumens gegen den ergonomischen Bereich 362 die für ein ausreichendes Haltern des Speichermediums (in den Figuren nicht gezeigt) zwischen der Anlagefläche 310 und der Klemmfläche 314 notwendige Haltekraft aufbringen.

In einem weiteren Unterschied zu der Ausführungsform der Figuren 1- 8 ist die Anlagefläche 310 nicht mittig zu der Achse A angeordnet, entlang derer sich der Aufbissabschnitt 308 erstreckt und entlang derer auch die Schiebebewegung des Schiebeteils 304 gegenüber dem Basisteil 302 stattfindet. Das Gleiche gilt für die Klemmfläche 314. Vielmehr sind Anlagefläche 310 und Klemmfläche 314 seitlich versetzt zu dieser Längsachse A angeordnet. Diese Anordnung ist vorteilhaft für Speichermedien, welche Verbindungsleitungen für beispielsweise eine Spannungsversorgung oder zur Übertragung von Daten aufweisen. Das Speichermedium, insbesondere ein Speichersensor, kann in diesem Fall so in den Halterungsabschnitt 306 eingebracht werden, dass diese Verbindungsleitungen entlang der Längsachse A im unteren Bereich des Aufbissabschnitt 308 beispielsweise in entsprechend angebrachte Kabelclips oder Kabelklemmen (nicht abgebildet) eingeschoben werden können. Die seitliche Anordnung von Anlagefläche 310 und Klemmfläche 314 gibt hierfür genügend Raum für das Speichersensor eigene Kabelmanagement.

In gleicher Weise wie die erste Ausführungsform sind sowohl in dem Basisteil 302 Öffnungen 330, 332 in dem Arretierbereich 324 des Basisteils 302 als auch Öffnungen 334, 336 (in Figur 11 ist nur die Öffnung 336 erkennbar) in dem Arretierbereich des Schiebeteils 304 vorgesehen. In diese Öffnungen 330-336 kann in gleicher Weise wie in der ersten Ausführungsform eine Ziel- oder Führungsstange 150 eingebracht werden, um Basisteil 302 und Schiebeteil 304 miteinander in einer Klemmstellung zu arretieren bzw. zu verriegeln.

Zusätzlich oder alternativ kann dieses Verriegeln bzw. Arretieren in der Klemmstellung auch durch einen Rastmechanismus zwischen Basisteil 302 und Schiebeteil 304 wie bereits bei der ersten Ausführungsform beschrieben realisiert werden (in den Figuren 11 und 12 nicht dargestellt). Dieses Verrasten kann durch geeignete Bedienelemente des Rastmechanismus für eine Bedienperson wieder lösbar sein, so dass Basisteil 302 und Schiebeteil 304 beispielsweise für eine Sterilisation trennbar sind.

## Patentansprüche

1. Vorrichtung zur Halterung einer Speicherfolie in der Mundhöhle eines Patienten für wenigstens eine Röntgenaufnahme, wobei die Vorrichtung einen Halteabschnitt und einen Aufbissabschnitt, ein erstes Teil mit einem Anlagebereich mit einer eben ausgebildeten Anlagefläche für die Speicherfolie,
einem ersten Führungs- oder Gelenkbereich sowie einem ersten Arretierbereich und ein zweites Teil mit einem Klemmbereich für die Speicherfolie, wobei der Klemmbereich bezüglich der Speicherfolie eine konvex geformte Fläche aufweist, einem mit dem ersten Führungs- oder Gelenkbereich korrespondierenden zweiten Führungs- oder Gelenkbereich sowie einem zweiten Arretierbereich aufweist, wobei der Anlagebereich und der Klemmbereich zusammenwirken und den Halteabschnitt bilden, der erste und der zweite Führungs- oder Gelenkbereich so zusammenwirken, dass das erste Teil und das zweite Teil relativ zueinander zwischen einer Offenstellung und einer Klemmstellung bewegbar sind und in der Klemmstellung zwischen dem Anlagebereich und dem Klemmbereich eine Klemmkraft auf ein dazwischen befindliche Speicherfolie ausübbar ist, wobei das erste Teil und das zweite Teil in der Klemmstellung über ein Zusammenwirken des ersten und des zweiten Arretierbereichs zueinander arretierbar sind.

2. Vorrichtung nach Anspruch 1, wobei die Anlagefläche zur flächigen Abstützung einer Speicherfolie ausgebildet ist.

3. Vorrichtung nach Anspruch 1 oder 2, wobei die Anlagefläche in einem Winkel zwischen 75° und 95°, bevorzugt zwischen 80° und 90°, zu dem Aufbissabschnitt angeordnet oder/und einstellbar ist.

4. Vorrichtung nach einem der Ansprüche 1 bis 3, wobei der Halteabschnitt in Bezug auf den Aufbissabschnitt in seiner Neigung verstell- und arretierbar ist.

5. Vorrichtung nach Anspruch 4, wobei die Neigung in einem Winkel zwischen 75° und 95°, bevorzugt zwischen 80° und 90°, zu dem Aufbissabschnitt angeordnet oder/und einstellbar ist.

6. Vorrichtung nach einem der Ansprüche 1 bis 5, wobei an dem ersten Teil und dem zweiten Teil in dem Arretierbereich miteinander korrespondiere Ausnehmungen so angeordnet sind, dass durch die Ausnehmungen eine Arretiervorrichtung steckbar und so das erste Teil und das zweite Teil miteinander arretierbar sind.

7. Vorrichtung nach einem der Ansprüche 1 bis 6, wobei der Führungsbereich eine Leisten-Führung aufweist, die so ausgestaltet ist, dass der Anlagebereich und der Klemmbereich zueinander von einer geöffneten Stellung in eine geschlossene Stellung verschiebbar sind und so die Klemmkraft von dem Klemmbereich auf den Anlagebereich ausübbar ist.

8. Vorrichtung nach einem der Ansprüche 1 bis 7 mit einem Gelenkbereich, wobei der Gelenkbereich an dem ersten oder dem zweiten Teil eine Drehachse und eine dazu korrespondierende Lagerung an dem anderen Teil aufweist.

9. Vorrichtung nach Anspruch 8, wobei die Drehachse so angeordnet ist, dass der Anlagebereich und der Klemmbereich eine Schwenkbewegung zueinander ausführen können.

10. Vorrichtung nach einem der Ansprüche 1 bis 9, wobei zwischen dem Anlagebereich und dem Klemmbereich ein Abstand vorhanden ist, der so dimensioniert ist, dass in der Offenstellung die Speicherfolie zwischen dem Anlagebereich und dem Klemmbereich eingebracht werden kann und in der Klemmstellung eine vorbestimmte Klemmkraft auf die Speicherfolie ausgeübt wird, die so dimensioniert ist das, dass bei einer normalen Handhabung der Vorrichtung die Speicherfolie festgeklemmt ist.

11. Vorrichtung nach einem der Ansprüche 1 bis 10, wobei der Anlagebereich in seinem unteren Bereich einen konkav geformten Übergangsabschnitt an oder/und ausgeformt hat.

## Claims

1. A device for holding an imaging plate in an oral cavity of a patient for at least one X-ray exposure, the device comprising a holding portion and a bite portion, a first part with an abutment region having a planar abutment surface for the imaging plate, a first guide or joint region and a first locking region, and a second part with a clamping region for the imaging plate, wherein the clamping region has a convex-shaped surface with respect to the imaging plate, a second guide or joint region corresponding to the first guide or joint region as well as a second locking region, wherein said abutment region and said clamping region cooperate to form the holding portion, said first guide and joint region and said second guide and joint region cooperate such that said first part and said second part are movable relative to each other between an open position and a clamping position, and, in the clamping position, a clamping force can be applied to an imaging plate located between the abutment region and the clamping region, wherein the first part and the second part are lockable relative to each other in the clamping position via a cooperation of the first and the second locking regions.

2. The device according to claim 1, wherein the abutment surface is configured for flat support of an imaging plate.

3. The device according to claim 1 or 2, wherein the abutment surface is arranged or/and adjustable at an angle between 75° and 95°, preferably between 80° and 90°, with respect to the bite portion.

4. The device according to any one of claims 1 to 3, wherein the holding portion is adjustable and lockable in its inclination with respect to the bite portion.

5. The device according to claim 4, wherein the inclination is arranged or/and adjustable at an angle between 75° and 95°, preferably between 80° and 90°, to the bite portion.

6. The device according to any one of claims 1 to 5, wherein recesses corresponding to one another are arranged on the first part and the second part in the locking region in such a way that a locking device can be inserted through the recesses and thus the first part and the second part are configured to be locked to one another.

7. The device according to any one of claims 1 to 6, wherein the guide region comprises a ledge guide configured to allow the abutment region and the clamping region to slide relative to each other from an open position to a closed position and thus the clamping force can be applied from the clamping region to the abutment region.

8. The device according to any one of claims 1 to 7 having wherein a joint region, wherein the joint region has an axis of rotation on the first or the second part and a bearing corresponding thereto on the other part.

9. The device according to claim 8, wherein the axis of rotation is arranged such that the abutment region and the clamping region are configured to perform a pivoting movement relative to each other.

10. The device according to any one of claims 1 to 9, wherein between the abutment region and the clamping region there is a distance dimensioned such that in the open position the imaging plate can be inserted between the abutment region and the clamping region and in the clamping position a predetermined clamping force is exerted on the imaging plate dimensioned such that during normal handling of the device the imaging plate is clamped.

11. The device according to any one of claims 1 to 10, wherein the abutment region has a concave-shaped transition section at or/and formed in its lower region.

## Revendications

1. Dispositif pour maintenir une plaque d'imagerie dans la cavité buccale d'un patient pour au moins une radiographie, dans lequel le dispositif comprend une section de maintien et une section à mordre, une première partie comportant une zone d'appui avec une surface d'appui plane pour la plaque d'imagerie, une première zone de guidage ou d'articulation ainsi qu'une première zone de blocage et une deuxième partie comportant une zone de serrage pour la plaque d'imagerie, la zone de serrage comprenant une surface de forme convexe par rapport à la plaque d'imagerie, une deuxième zone de guidage ou d'articulation correspondant à la première zone de guidage ou d'articulation, ainsi qu'une deuxième zone de blocage, la zone d'appui et la zone de serrage coopérant et formant la section de maintien, la première et la deuxième zone de guidage ou d'articulation coopérant de telle sorte que la première partie et la deuxième partie sont mobiles l'une par rapport à l'autre entre une position ouverte et une position de serrage et que, dans la position de serrage, une force de serrage peut être exercée entre la zone d'appui et la zone de serrage sur une plaque d'imagerie se trouvant entre celles-ci, dans lequel la première partie et la deuxième partie peuvent être bloquées l'une par rapport à l'autre dans la position de serrage par une coopération entre la première et la deuxième zone de blocage.

2. Dispositif selon la revendication 1, dans lequel la surface d'appui est conçue pour soutenir à plat une plaque d'imagerie.

3. Dispositif selon la revendication 1 ou 2, dans lequel la surface d'appui est disposée et/ou réglable à un angle compris entre 75° et 95°, de préférence entre 80° et 90°, par rapport à la section à mordre.

4. Dispositif selon l'une quelconque des revendications 1 à 3, dans lequel la section de maintien peut être réglée et bloquée dans son inclinaison par rapport à la section à mordre.

5. Dispositif selon la revendication 4, dans lequel l'inclinaison peut être disposée et/ou réglée à un angle compris entre 75° et 95°, de préférence entre 80° et 90°, par rapport à la section à mordre.

6. Dispositif selon l'une quelconque des revendications 1 à 5, dans lequel des évidements correspondant les uns aux autres sont disposés sur la première partie et la deuxième partie dans la zone de blocage, de telle sorte qu'un dispositif de blocage peut être inséré à travers les évidements et qu'ainsi la première partie et la deuxième partie peuvent être bloquées l'une avec l'autre.

7. Dispositif selon l'une quelconque des revendications 1 à 6, dans lequel la zone de guidage comprend un guidage à rainure qui est conçu de telle sorte que la zone d'appui et la zone de serrage peuvent être déplacées l'une par rapport à l'autre d'une position ouverte à une position fermée et qu'ainsi la force de serrage peut être exercée par la zone de serrage sur la zone d'appui.

8. Dispositif selon l'une quelconque des revendications 1 à 7, comportant une zone d'articulation, dans lequel la zone d'articulation comprend un axe de rotation sur la première ou la deuxième partie et un palier correspondant sur l'autre partie.

9. Dispositif selon la revendication 8, dans lequel l'axe de rotation est disposé de telle sorte que la zone d'appui et la zone de serrage peuvent effectuer un mouvement de pivotement l'une par rapport à l'autre.

10. Dispositif selon l'une quelconque des revendications 1 à 9, dans lequel il existe entre la zone d'appui et la zone de serrage un écart dimensionné de telle sorte que, dans la position ouverte, la plaque d'imagerie peut être introduite entre la zone d'appui et la zone de serrage et que, dans la position de serrage, une force de serrage prédéterminée est exercée sur la plaque d'imagerie, laquelle est dimensionnée de telle sorte que, lors d'une manipulation normale du dispositif, la plaque d'imagerie est fermement serrée.

11. Dispositif selon l'une quelconque des revendications 1 à 10, dans lequel la zone d'appui comprend dans sa partie inférieure une section de transition de forme concave rapportée et/ou moulée.
